# EUROPEAN PATENT SPECIFICATION

(11) **EP 1 737 369 B1**
(45) Date of publication and mention of the grant of the patent: **13.05.2009**
(21) Application number: 05735303.9
(22) Date of filing: 14.04.2005
(51) Int. Cl.: A61B 17/80

(54) **SUPPORT FOR BONE**
KNOCHENSTÜTZE
SUPPORT POUR OS

(30) Priority: 16.04.2004 US 826684; 20.12.2004 US 19918
(43) Date of publication of application: 03.01.2007
(73) Proprietor: Kyphon SÀRL, 2000 Neuchâtel (CH)
(72) Inventor: MALANDAIN, Hugues, F., Mountain View, CA 94043 (US)
(74) Representative: Wombwell, Francis
(86) International application number: PCT/US2005/012736
(87) International publication number: WO 2005/104971

(56) References cited:
- WO-A-00/54681
- FR-A- 2 780 631
- US-A1- 2002 026 194
- US-A1- 2003 135 210

## Description

### TECHNICAL FIELD

This invention relates to medical devices.

### BACKGROUND

The use of spinal stabilization/fixation devices to align position, and/or mechanically stabilize specific vertebrae or a region of the spine is well established. Typically such devices utilize a spinal fixation element, comprised of a relatively rigid member such as a plate, board or rod, which is used as a coupler between adjacent vertebrae. Such a spinal fixation element can effect a rigid positioning of adjacent vertebrae when attached to the pedicle portion of the vertebrae using pedicle bone anchorage screws. Once the coupled vertebrae are spatially fixed in position, procedures can be performed. heating can proceed or spinal fusion may take place.

Spinal fixation elements may be introduced posteriorly to stabilize the various vertebrae of the spine, for example, in conjunction with a kyphoplasty procedure wherein a void or cavity is made inside a vertebral body followed by filling with a bone substitute to form an "internal cast." Some conventional devices for this purpose are designed to be attached directly to the posterior of the spine, but the generally invasive nature of a conventional posterior approach used to implant these devices poses drawbacks. One minimally invasive solution to the problem of the posterior approach involves making a longitudinal separation of the sacrospinalis group between the multifudus and longissimus utilizing the natural cleavage plane between these two muscle rather than detaching the paraspinal muscles from the posterior spinal elements. Problems stemming from the prior art solutions include a high degree of invasiveness resulting in muscle disruption and blood loss. The loss of the paraspinal muscle attachment sites, formation of scar tissue, and loss of muscle function may compromise the patient's final outcome. Additionally, the prior art solutions are time consuming and are difficult to remove.
US Patent No. US2002026194 describes a multi-axial bone fixation implant. The preamble of claim 1 is based on this document.

### SUMMARY

In general, in one aspect, the invention provides an implantable medical device for supporting a structure according to claim 1.

Aspects of the invention provide numerous advantages.

the structure supported by the device can be a bony structure. Alternatively, the structure supported can be the spine. In one implementation, the posterior of the spine can be the supported structure. In other implementations, other portions of the spine are the supported structure. In yet other implementations, the structure supported can include a femur or other bones of the leg (e.g. tibia and fibula), bones of the arm and wrist (e.g. humerus, radius and ulna), calcaneous, pelvis, spine and the like.

The base of the anchor assembly of the device can be comprised of a base head that is movably disposed within the anchor assembly.

The support element can have one or more apertures with a dimensional configuration providing access to the base, as well as the means for locking the base to the anchor assembly, through the top portion of the support element. The support element apertures are configured such that the head of the base disposed within the anchor assembly does not pass through the support element. In one implementation, the support element of the device is elongated and can have a shape selected from the group consisting of a board, plate, elongated cross-section, oval, square, I-beam and a rod. Additionally, the support element can be sized to substantially span two or more vertebrae of the spine. The support element can be comprised of a material selected from the group consisting of titanium, stainless steel, carbon fiber, a biocompatible material, a reabsorbable material and composites thereof.

The receiver can be integrally disposed within the bottom surface of the bottom portion of the support element. In one implementation, the receiver can be attached to the bottom surface of the bottom portion of the support element. In another implementation, the receiver can have a configuration selected from the group consisting of a slot, groove, track, dovetail and one-way snap-in configuration. Additionally, the receiver can have a 90-degree twist-in configuration. Alternatively, the receiver and the anchor assembly can interconnect through a T-slot configuration. In the T-slot implementation, the receiver can include a planar upper face, a planar lower face and a planar medial face.

The receiver can substantially span the length of the bottom surface of the bottom portion of the support element, and have a plurality of ends. The receiver can have a first end that is open and a second end that is closed. Additionally, the receiver can have first and second ends that are both open. Alternatively, the receiver can have first and second ends that are both closed. The receiver can have a plurality of access ports sized for coupling the anchor assembly to the receiver distally from the receiver ends. Additionally, the receiver can be configured to receive the anchor assemblies in two dimensions.

The anchor assembly can be of a configuration selected from the group consisting of a slot, groove, track, dovetail and one-way snap-in configuration. The anchor assembly can have a 90-degree twist-in configuration or a T-slot configuration. The anchor assembly can be comprised of a material selected from the group consisting of titanium, stainless steel, carbon fiber, a biocompatible material, a reabsorbable material and composites thereof.

The means for locking the anchor assembly to the support element can be comprised of a setscrew disposed within a threaded anchor assembly locker aperture. The setscrew can be threaded so as to lockably engage the receiver planar upper face upon turning, such that upon so engaging the receiver planar upper face, the setscrew causes the anchor assembly to press against the receiver lower planar face to effect locking. Alternatively, the means for locking the anchor assembly to the support element can be a cam. The means for locking the anchor assembly to the support element can be comprised of a threaded blind aperture having a tapered slot substantially aligned longitudinally with the receiver thereby providing expandable walls, a floor having a channel cut therethrough and a setscrew. Turning the setscrew into the blind aperture causes the walls to expand outwardly to engage the receiver planar medial surface to effect locking. Alternatively, the blind aperture is configured for a cam to effect locking of the anchor assembly to the support element in a manner analogous that described for a setscrew.

The base of the anchor assembly can be selected from the group consisting of a screw, staple, nail, hook and a pin. The screw can be a bone anchorage screw. The bone anchorage screw can be a pedicle screw. The base head of the anchor assembly base can be polyaxial or a hinge-type connector. The base can have a means for locking the base in a desired position. The means for locking the base in position can be comprised of a threaded base aperture and a setscrew; wherein turning the setscrew into the threaded base aperture results in engagement of the base head to effect locking. Alternatively, the means for locking the base is a cam wherein the cam is disposed such that turning the cam results in engagement of the base head with the cam to effect locking.

A method for supporting a spine using one aspect of the device can include the steps of: implanting a plurality of anchor assemblies having bases into the pedicles of adjacent vertebrae of a spine; connectively positioning a support element having a receiver for the anchor assemblies on top of the anchor assemblies; locking the bases within the anchor assemblies; and locking the anchor assemblies within the support element receiver. In one implementation, the support element can be disposed within a body location selected from the group consisting of the subcutaneous fat layer of the back, muscle, cartilage and a bone. In another implementation, the support element is disposed adjacent to bone. In yet another implementation, the support element is disposed adjacent to the spine. In another implementation, the support element is disposed external to the body.

A method of use of the device according to the invention for effecting a desired vertebral disk spacing, can include the steps of: 1) implanting the bases of a plurality of anchor assemblies into vertebrae, wherein the bases of the anchor assemblies are unlocked for free movement within the anchor assemblies; 2) interconnecting the anchor assemblies with a receiver of the support element, wherein the anchor assemblies are unlocked within a receiver; 3) locking the bases within the anchor assemblies using a setscrew or cam; 4) compressing or distracting the bases in relation to each other to achieve a parallel displacement of the instrumented vertebrae; and 5) locking the anchor assemblies within the support element using a set screw or cam.

A method of use of the device according to invention for effecting a desired curvature of the spine can include the steps of: 1) implanting the bases of a plurality of anchor assemblies into vertebrae, wherein the bases of the anchor assemblies are unlocked for free movement; 2) interconnecting the anchor assemblies with a receiver of a support element, wherein the anchor assemblies are unlocked within the receiver; 3) compressing or distracting the bases in relation to each other to affect the lordotic/kyphotic curvature of the spine; 4) locking the bases within the anchor assemblies and locking the anchor assemblies within the support element, using a setscrew or cam.

Another method of using the device according to the invention to support the spine can include the steps of: 1) setting a series of anchor assemblies percutaneously in place along the spine through a series of small incisions including screwing a bone anchorage screw of each anchor assembly into one or more adjacent pedicle portions of adjacent vertebrae in the spine, such that the anchor assemblies' receiver mating parts align in a parallel plane within the subcutaneous fat layer of the back; 2) loading a support element on top of the anchor assemblies including engaging the mated parts of the receiver and the anchor assembly, either by sliding, snapping or otherwise positioning the support element into the desired position; 3) accessing and locking the anchor assembly in the support element using the locking means feature of the anchor assembly via the support element apertures; and 4) optionally locking the bone anchorage screw feature of the anchor assembly using the locking means feature for the bone anchorage screw via the support element apertures.

In general, in another aspect, a minimally invasive, low profile implant device for supporting the spine is provided. In general, in one aspect an implantable medical device is provided useful for supporting a body structure, such as the spine, using a percutaneously disposed support element combined with a plurality of anchor assemblies having movably attached bases. Each base can be a bone anchorage screw. Alternatively, each base is a bone staple, nail, hook or screw. The assembled support element and anchor assembly provide support to the spine without the need for passage of a bone anchorage screw through the support element. The base features of the anchor assemblies can be of a type used for insertion into the pedicles of vertebrae. The support element is configured for mated placement on top of a number of anchor assemblies and can be locked in place. Locking of the anchor assemblies within the support element is effected through the top of the support element via apertures passing through the support element. The bone anchorage screw feature can optionally be locked to prevent movement within the anchor assembly to further support the spine.

In general, in another aspect, an implantable medical device is provided for supporting a structure including a support having a top and bottom portion and one or more apertures passing therethrough. The bottom portion is adapted to receive a receiver. The receiver includes one or more anchor assemblies, each including a head and a base portion. The base portion engages the structure to be supported, and the receiver engages the bottom portion of the support. When the receiver is assembled with the support, the base does not pass through the one or more apertures. The receiver further includes a second anchor assembly passable through one of the apertures of the receiver and lockably engagable with the support.

Implementations of the invention can include one or more of the following features. The base head can be movably disposed within the anchor assembly. The base can further include a locking means for locking the base in a desired position relative to the support.

The one or more apertures of the device can have a dimensional configuration providing access to a base and the means for locking an anchor assembly when assembled with the support.

The support of the device can have a shape selected from the group consisting of a board, plate, elongated cross-section, oval, square, I-beam and a rod. The support bottom portion can include a bottom surface, wherein the receiver is coupled to the bottom surface through a means including integral attachment and non-integral attachment.

The receiver and the anchor assembly of the device can be configured in an interconnecting geometry including a T-slot having a planar upper face, a planar lower face and a planar medial face. The receiver can include one or more access ports sized for coupling an anchor assembly to the receiver distally from the receiver ends.

The head of the one or more anchor assemblies and the second anchor assembly can include a slot, groove, track, dove tail and a snap-in configuration.

The base of the device can be a screw, staple, nail, hook or a pin. In one embodiment, the base is a screw that is a bone screw. The base head of the base can include a polyaxial and a hinge-type connector.

The means for locking the anchor assemblies to the support can include a deformable geometry of the head portion of the one or more anchor assemblies. The deformable geometry of the head can include a tapered void within the head, and a setscrew. Turning the setscrew into the threaded base aperture can result in deformation of the head outwardly, and thereby cause the head to engage the receiver to effect locking.

The means for locking the base can include a threaded base aperture and a setscrew. Turning the setscrew into the threaded base aperture can result in engagement of the base head to effect locking the base in a desired position.

The structure supported by the device can be a fractured bony structure.

Aspects of the invention may include one or more of the following advantageous features. In various implementations of the invention wherein the support top portion has a smooth surface with no protruding features (e.g., screw heads or the like), such is desirable and advantageous since the smooth surface limits irritation of tissues and presents a smooth profile under the skin. In one implementation a smooth profile is achieved wherein the base head of the base, though accessible through the support, does not pass through the support or protrude above the top portion. In another implementation, a smooth profile is achieved wherein the support apertures are sized such that the anchor assembly can pass completely through the support top portion or bottom portion and be lockably engaged within the support without protruding from the support.

Placement of the support can be in the subcutaneous fatty layer of the back. Such placement is desirable since less tissue irritation, discomfort and scarring will be experienced by a patient. Additionally, when a temporary support is indicated, such placement allows for easy removal of the support device once healing or treatment is complete (e.g., when a fractured or diseased vertebral body has been healed or treated).

A two-step independent locking of the anchor assemblies to the support and of the bases to the anchor assemblies can be provided. One type of anchor assembly can be constructed and arranged for loading and locking into a receiver by way of the bottom surface of the bottom portion of the support. These anchor assemblies include a first locking means for securing the anchor assembly within the support, and a second locking means for securing the base within the anchor assembly. In this implementation, the bases when loaded into the anchor assembly do not pass through the support, but can be accessed through an aperture in the support from above. Such an arrangement allows for a two-step independent locking process wherein either the anchor assembly is first locked to the support or the base is first locked to the anchor assembly. By selecting which feature is locked first, various procedures and maneuvers can be performed upon the structure(s) to which the bases are attached (e.g., manipulating vertebral body spacing, adjusting spinal curvature or both).

In certain implementations of the invention where three anchor assemblies having bases are provided, two anchor assemblies flanking a third central assembly can be manipulated to effect spacing and curvature of the spine and to hold the position of the spine around the spinal structure (e.g., vertebral body, annulus fibrosis or nucleus pulposus) to be treated. Correction maneuvers can be performed prior to, or after placement of, the central anchor assembly and base depending on the nature of the procedure to be performed. It should be understood that the spinal structure (vertebra or spinal disc) can be treated with or without the central anchor assembly in position, and with or without the support in position. If the support is in position, treatment can occur using instruments that pass through the aperture of the support. Treatment can also occur through the longitudinal aperture of any of the base(s). Another aspect of this implementation is the ease of insertion of the third anchor assembly through the top of the support. Additional surgical skill is required to align and attach a third anchor assembly in the receiver of the support, even more so than to align and attach two flanking anchor assemblies. In this implementation, the flanking anchor assemblies are positioned first in the receiver, and the central anchor assembly is positioned last and passes through the top of the support.

Methods using permutations and combinations of the two types of anchor assembly described herein are desirable for use in supporting structures. It is understood that a number of combinations of the two anchor assembly types including a support having two or three anchor assemblies are contemplated and are part of this disclosure. Included in the possible permutations of use are various alternative ordered steps for locking the anchor assemblies within the support and for locking the base within the anchor assembly.

In implementations of the invention wherein the anchor assembly includes a longitudinal aperture through the base and base head, the longitudinal aperture and setscrew aperture can be constructed and arranged for delivery of a bone substitute into the bone to be treated. Such an implementation allows for percutaneous placement of the base using imaging guidance such as fluoroscopy, and for delivery of bone filler material.

The details of one or more embodiments of the invention are set forth in the accompanying drawings and the description below. Other features, objects, and advantages of the invention will be apparent from the description and drawings, and from the claims.

### DESCRIPTION OF DRAWINGS

FIG. 1 is a drawing of the device showing three anchor assemblies positioned within the support element.

FIG 2A is a drawing showing a top view of the support element, illustrating the receiver and apertures.

FIG 2B is a drawing showing a bottom view of the support element, illustrating the receiver and apertures.

FIG. 2C is a drawing showing an end view of the support element, illustrating the receiver.

FIG. 2D is a drawing showing two bottom views of the support element, illustrating the anchor assembly access ports.

FIG. 3A is a drawing showing a side view of the anchor assembly, illustrating the base, the locker aperture and the base aperture.

FIG. 3B is a drawing showing a bottom view of the anchor assembly, illustrating the base and the threaded anchor assembly locker aperture.

FIG. 3C is a drawing showing a cutaway view of the anchor assembly, illustrating the base head, the receptacle, the base aperture and the setscrew.

FIG. 4A is a drawing showing the locker aperture and setscrew means for locking the anchor assembly to the support element.

FIG 4B is a drawing showing two views of a threaded blind aperture with slot and setscrew means for locking the anchor assembly to the support element.

FIG 4C is a drawing showing a screw, locker aperture and cavity means for locking the anchor assembly to the support element.

FIG. 5A is a drawing of a support device showing two different anchor assembly types positioned within the support.

FIG. 5B is a drawing showing a top view of the support, illustrating the receiver and apertures.

FIG. 5C is a drawing showing a bottom view of the support, illustrating the receiver and apertures.

FIG. 5D is a drawing showing a bottom view of the support, illustrating the anchor assembly access ports.

FIG. 5E is a drawing showing a cross-sectional end view of the support, illustrating the receiver.

FIG. 6 is a drawing of the device showing three anchor assemblies positioned within the support.

FIG 7 is a drawing of the device showing two anchor assemblies positioned within the support.

FIG 8A is a drawing showing a cutaway view of one anchor assembly type and the support, illustrating the receiver of the support, and the anchor assembly prior to locking into the support.

FIG 8B is a drawing showing a cutaway view of one anchor assembly type and the support, illustrating the receiver of the support, and the anchor assembly after locking into the support.

FIG 9 is a drawing showing a cutaway view of another anchor assembly type, illustrating the anchor assembly head, base, base head, longitudinal aperture, tool interface, receptacle, base aperture and setscrew.

Like reference symbols in the various drawings indicate like elements.

### DETAILED DESCRIPTION

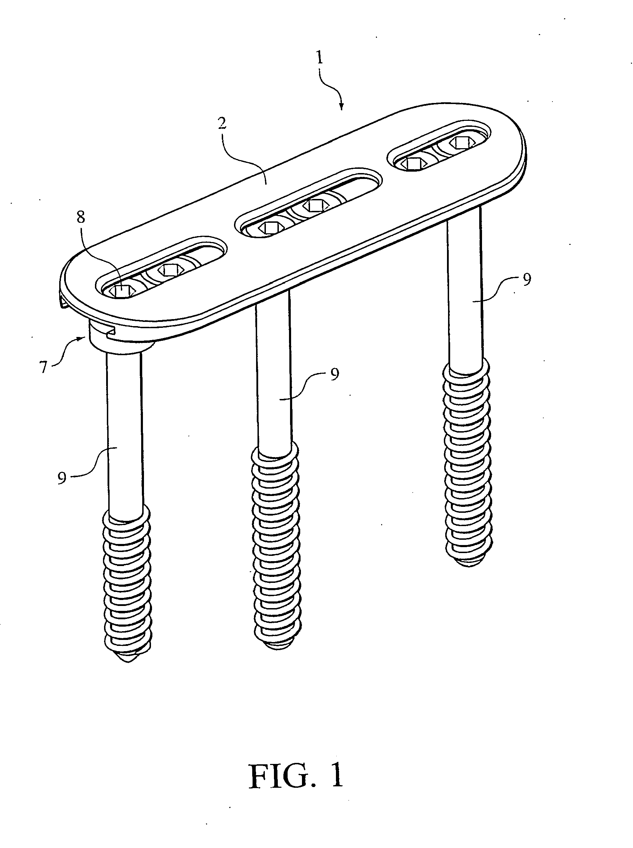

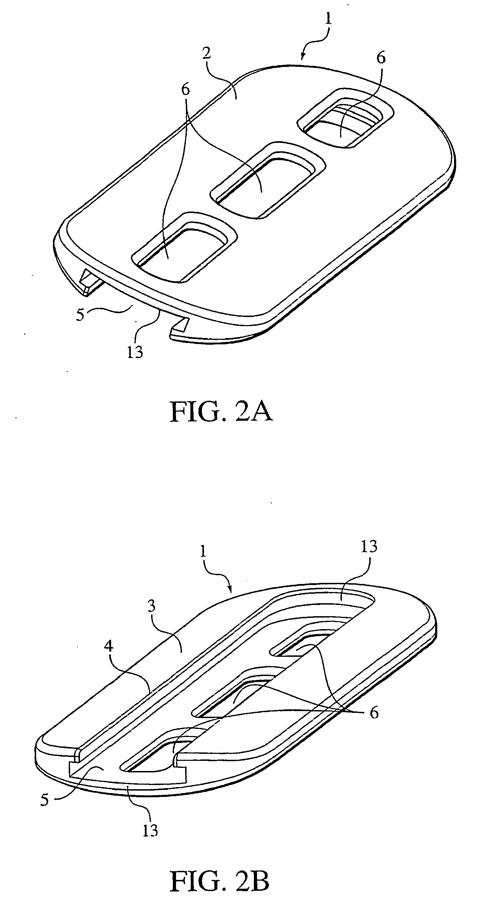

As shown in FIG 1 and FIGS. 2A-D, a support element 1 is provided having a shape. The support element 1 preferably is elongate and of a sufficient length to span a number of vertebrae in the spine. The support element 1 shape can include board, elongated cross-section, rod, oval, square, and I-beam. The length of the support element 1 is minimally substantially the same length as required to span two or more vertebrae. Preferably, the support element 1 is substantially the same length as required to span three vertebrae. In one implementation, the length of the support element 1 is substantially between 25 to 140 millimeters. The support element 1 can be made of materials that are durable and that can be implanted in a body, including titanium, stainless steel, carbon fiber, etc. In one implementation, the support element 1 is made of titanium. In another implementation the support element 1 is made of a biocompatible material. Additionally, the support element 1 can be made of a reabsorbable material. Furthermore, the support element can be made of a composite of any of the forgoing materials. In use, the support element 1 and attending anchor assemblies (described in detail below) can be used for temporary or permanent implantation.

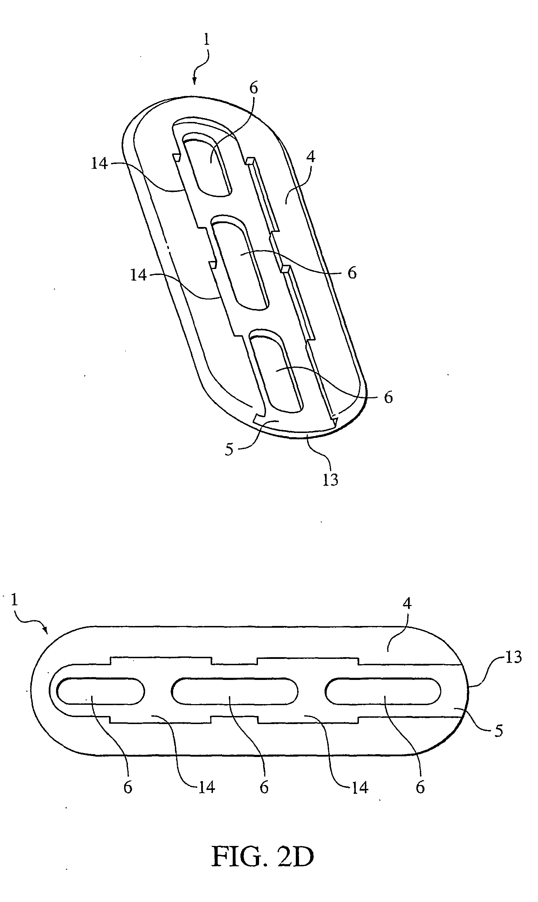

As shown in FIGS. 2A -D, the support element 1 includes a top portion 2 and a bottom portion 103 having a bottom surface 4 and a receiver 5. Additionally, as shown in FIGS. 2A and 2B, the support element 1 can include a number of centrally aligned apertures 6. The apertures 6 can have any of a number of shapes not limited to openings having a round, oval or elongate shape. Any number of apertures 6 can be employed, including one or more. In one implementation, three apertures 6 per support element 1 are included (See FIGS. 1, 2A, 2B and 2D). Any type of spacing of apertures 6 can be used, including even or staggered. In one implementation, the apertures 6 are spaced evenly and separated by a distance substantially equivalent to the spacing of the vertebrae of a spine. Alternatively, the apertures 6 can be spaced closer together than the spacing of vertebrae of a spine. The aperture(s) 6 are sized for and limited to a size sufficient for accessing the features of the anchor assembly 7 (described in detail below) with a tool or object through the top portion 2 of the support element 1 (e.g. to engage the locking means of the anchor assembly 7 using, for example, a hex-headed screw driver). In this way, as shown in FIG. 1, when the support element 1 and the anchor assembly 7 are assembled, the base head 8 of the base 9 (both described in detail below), though accessible through the support element 1, does not pass through the support element 1. In use (i.e., assembled with a plurality of anchor assemblies) the support element 1 top portion 2 can have a smooth surface with no protruding features (e.g., screw heads or the like).

In another implementation, the aperture(s) 6 are sized such that when the support element 1 and the anchor assembly 7 are assembled, the base head 8 portion of the base 9 can pass through the support element 1 (e.g., from beneath), but the base 9 does not pass through the support element 1 (not shown). In yet another implementation, the aperture(s) 6 are sized such that the base 9 can pass completely through the support element 1 (not shown).

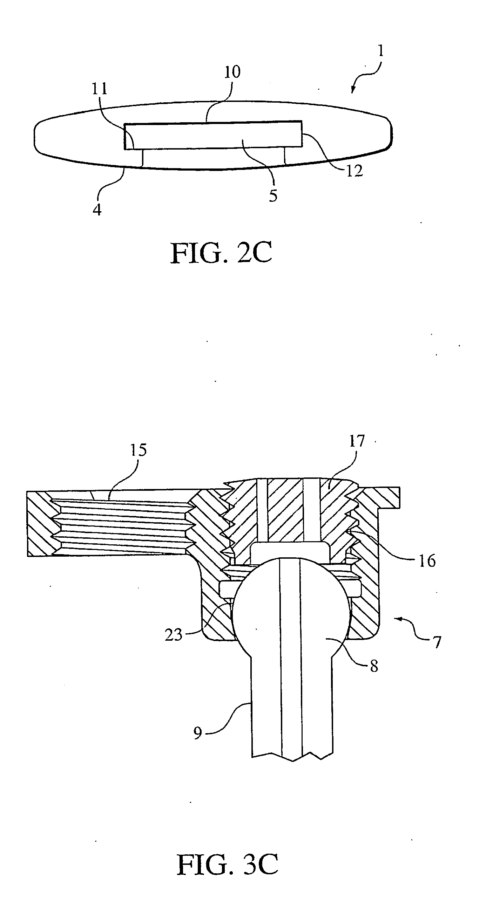

As shown in FIGS. 2A-D the support element 1 includes a receiver 5 that is integrally disposed within the bottom surface 4 of the support element 1. Alternatively, the receiver 5 can be coupled to the bottom surface 4 of the support element 1 (for example a track-type receiver 5 can be attached by rivets or screws to the bottom surface 4 of the support element 1). The receiver 5 can be configured as a slot, groove, track, dovetail, one-way snap-in design, or the like. In one implementation, the receiver 5 is configured in a 90-degree twist-in configuration, wherein the base head 8 of the anchor assembly 7 base 9 has two dimensions. The first dimension allows the base head 8 to pass into the receiver 5. As the base head 8 is rotated 90 degrees with respect to the receiver 5 and, upon completing the 90-degree rotation, the second dimension maintains the base 9 in the receiver 5. In another implementation, as shown in FIGS. 2A-C, the receiver 5 is a T-slot configuration. As shown in FIG. 2C, the receiver 5 includes a planar upper face 10, a planar lower face 11 and a planar medial face 12. Alternatively, where the configuration of the receiver 5 is dovetail, the receiver 5 includes a planar upper face 10 and an angled face. In yet another alternative, where the receiver 5 is of a curved or rounded shape, the receiver 5 includes a curved or rounded receiver 5 face. The receiver 5 can span all or part of the length of the bottom surface 4 of the support element 1. As shown in FIGS. 2A, 2B and 2D, each end 13 of the receiver 5 can be open or closed. In one implementation, both ends 13 of the receiver 5 are open (not shown). In another implementation, one end 13 of the receiver 5 is open and the opposite end 13 is closed (see FIGS. 2B and 2D). In another implementation, both ends 13 of the receiver 5 are closed (not shown).

As shown in FIG. 2D, the receiver 5 can include anchor assembly access ports 14 that provide openings in the pathway of the receiver 5, whereby the anchor assemblies 7 can be interconnected to the support element 1 without having to traverse the length of the receiver 5 when accommodating multiple (or even a single) anchor assemblies 7. The anchor assembly access ports 14 can be of a sufficient size to accept the anchor assembly 7 and can be spaced evenly, or staggered as desired.

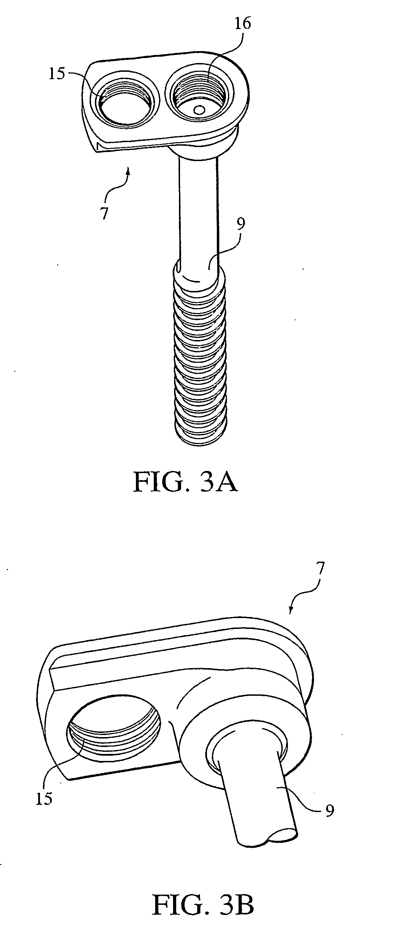

As shown in FIGS. 2A-C, 3A, 3B and 4A, the anchor assembly 7 is configured to interconnect with the shape of the receiver 5. As described above for the receiver 5, the anchor assembly 7 complimentary shapes can be any of a number of shapes. In some implementations, the mating between the support element 1 and the anchor assembly 7 occurs only in two dimensions (e.g. where a 90 degree twisting receiver 5 is employed). As shown in FIGS. 3A and 3B, the anchor assembly 7 can be comprised of a threaded anchor assembly 7 locker aperture 15 and a threaded base aperture 16. The anchor assembly 7 can be comprised of numerous materials that are durable and that can be implanted in a body, including titanium, stainless steel, carbon fiber, etc. Additionally, the anchor assembly 7 can be comprised of a reabsorbable material or a biocompatible material. Furthermore, the anchor assembly can be made of a composite of any of the forgoing materials.

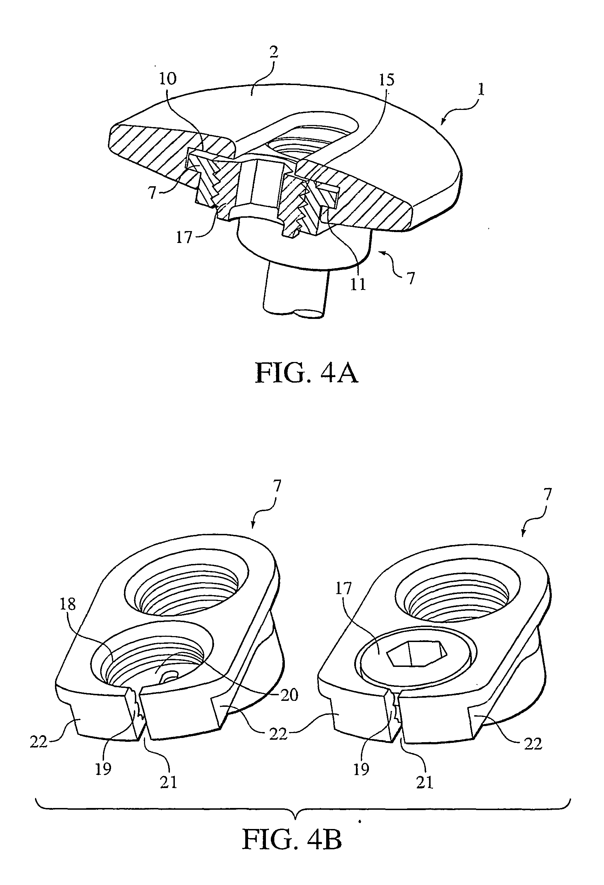

As shown in FIGS. 4A- 4C, the anchor assembly 7 includes a means for locking the anchor assembly 7 to the support element 1. In one implementation, as shown in FIG 4A, where the receiver 5 and complimentary anchor assembly 7 are T-slot shaped, the means for locking the anchor assembly 7 can be a setscrew 17 disposed within a threaded anchor assembly 7 locker aperture 15; wherein turning the setscrew 17 causes the setscrew 17 to move up toward the top portion 2 of the support element 1, where the setscrew 17 engages the receiver 5 planar upper face 10. When the setscrew 17 engages the receiver 5, the anchor assembly 7 presses against the receiver 5 planar lower face 11 to effect locking (See FIGS 2C and 4A). Alternatively, a cam can be substituted for the setscrew 17 to effect the locking of the anchor assembly 7 within the support element 1.

In a further implementation, as shown in FIG. 4B, where the receiver 5 and complimentary anchor assembly 7 are T-slot shaped, the means for locking the anchor assembly 7 includes a threaded blind aperture 18 having a tapered slot 19 substantially aligned with the receiver 5 path. The threaded blind aperture 18 terminates in a floor 20 with a channel 21 cut therethrough. The threaded blind aperture 18 and a setscrew 17 are sized such that when engaged (i.e., screwed downward), the setscrew 17 causes expansion of the anchor assembly 7 walls 22 outwardly, whereupon contact is made between the anchor assembly 7 walls 22 and the receiver 5 medial surface to effect locking. Alternatively, a cam can be substituted for the setscrew 17 to effect the locking of the anchor assembly 7 within the support element 1.

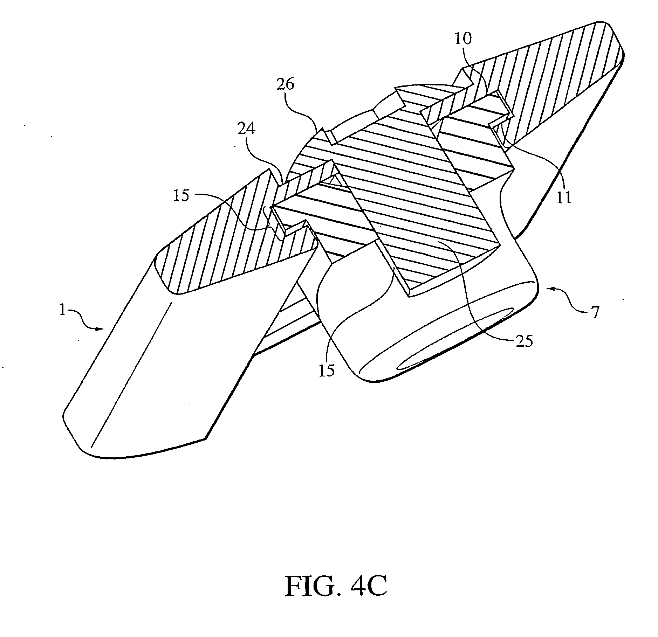

In another implementation, as shown in FIG. 4C, where the receiver and complimentary anchor assembly 7 are T-slot shaped, the means for locking the anchor assembly 7 includes a locker aperture 15 having a screw 25, cam or the likes which can be lockably connected to the anchor assembly 7. Additionally, in this implementation the top portion 2 of support element 1 includes a cavity 24 or recess, substantially aligned with the receiver 5 for accommodating a screw head 26 or cam. In use, when the screw head 26 of the screw 25 engages the cavity 24 of the receiver 5, the anchor assembly 7 is drawn against the receiver 5 planar upper face 10 to effect locking (See FIGS 2C and 4C). Alternatively, a cam can be substituted for the screw 25 to effect the locking of the anchor assembly 7 within the support element 1. In another implementation, the screw head 26 is configured as a button head style (see FIG. 4C) providing a low profile within the cavity 24 such that the screw head 26, can be positioned within the support element 1 without protruding above the top portion 2.

In other implementations where the receiver 5 and complimentary anchor assembly 7 are of alternative shapes or configurations (e.g. dove tail or rounded), an analogous means of locking is provided.

As shown in FIG. 3C, the anchor assembly 7 includes a base 9 moveably disposed within the threaded base aperture 16. The base 9 can be a screw, staple, hook or nail and of a type typically used for anchoring to a structure (e.g., a bone). In one implementation, the base 9 is screw of a type for insertion into the pedicle of a vertebrae. In another implementation, the base can be attached to another bony structure.

Attachment of the base 9 to the anchor assembly 7 can be made in any of a number of ways. In one implementation, the attachment is through a hinge-type of connection between the base 9 and the anchor assembly 7. In another implementation, as shown in FIG. 3C, the attachment is made between a polyaxial-type base head 8 on the base 9 and a complimentary receptacle 23 within the anchor assembly 7.

The anchor assembly 7 further includes a means for locking the base 9 within the anchor assembly 7. As shown in FIG. 3C, for a polyaxial-type base head 8, the means for locking can include a setscrew 17 disposed within the threaded base aperture 16. In this configuration, turning the setscrew 17 causes the setscrew 17 to press directly against the polyaxial head, thereby forcing the polyaxial head against the receptacle 23 of the anchor assembly 7 to effect locking. Alternatively, where the base 9 is of the hinge-type, the means for locking could be comprised of a setscrew 17 disposed in a threaded base aperture 16. In this configuration, turning the setscrew 17 causes the setscrew 17 to press directly against the base head 8 of the hinge-type base 9, thereby creating friction against the hinge's pin to effect locking (not shown). In another implementation, a cam can be substituted for the setscrew 17 to effect locking.

Support element 1 and a one or more anchor assemblies 7, once assembled, can be used to support a bony structure. When mated, the support element 1 and one or more anchor assemblies 7 form a support assembly. The bony structure supported can include a femur or other bones of the leg (e.g. tibia and fibula), bones of the arm and wrist (e.g. humerus, radius and ulna), calcaneous, pelvis, spine and the like. Support can be provided for a single bone (i.e. a long bone such as the femur, tibia, humerus) or for more than one bone (i.e. vertebrae).

In use, the support assembly can support a bony structure wherein the support element 1 is disposed within a body location including the subcutaneous fat layer of the back, muscle, cartilage, bone and the like. Alternatively the support element 1 is disposed adjacent to bone. In another implementation, the support element 1 is disposed external to the body.

Additionally, the support assembly includes a freedom of movement with regard to the base 9 within the anchor assembly 7 and the anchor assembly 7 within the support element 1. That is, prior to locking respective base 9 and the anchor assembly 7 elements of the support assembly (hereinafter referred to as an unlocked configuration), the elements of the support assembly are movable and have one or more degrees of freedom so as allow for movement of the underlying structure being supported. For example, in the unlocked configuration, the support assembly is configurable so as to facilitate manipulation of vertebral disk spacing and spine curvature. Since the base 9 and anchor assembly 7 can be tightened and loosened by independent locking means, an option is provided to increase or decrease the vertebral disk space/height, or to increase or decrease the amount or lordotic/kyphotic curve of the spine, also called curvatures of the spine.

A method of use of the device according to the for effecting a desired vertebral disk spacing, can include the steps of: 1) implanting the bases 9 of a plurality of anchor assemblies 7 into vertebrae, wherein the bases 9 of the anchor assemblies 7 are unlocked for free movement; 2) interconnecting the anchor assemblies 7 with the receiver 5 of the support element 1, wherein the anchor assemblies 7 are unlocked within the receiver 5; 3) locking the bases 9 within the anchor assemblies 7 using a setscrew 17 or cam; 4) compressing or distracting the bases 9 in relation to each other to achieve a parallel displacement of the instrumented vertebrae ("Instrumented" meaning where a physical connection exists between a structure (e.g. a vertebrae) and a medical device or instrument.); and 5) locking the anchor assemblies 7 within the support element 1 using a set screw 17 or cam.

A method of use of the device according to the invention for effecting a desired curvature of the spine can include the steps of: 1) implanting the bases 9 of a plurality of anchor assemblies 7 into vertebrae, wherein the bases 9 of the anchor assemblies 7 are unlocked for free movement; 2) interconnecting the anchor assemblies 7 with the receiver 5 of the support element 1, wherein the anchor assemblies 7 are unlocked within the receiver 5; 3) compressing or distracting the bases 9 in relation to each other to affect the lordotic/kyphotic curvature of the spine; 4) locking the bases 9 within the anchor assemblies 7 and locking the anchor assemblies 7 within the support element 1, using a setscrew 17 or cam.

Another method of using the device acording to the invention to support the spine can include the steps of: 1) setting a series of anchor assemblies 7 percutaneously in place along the spine through a series of small incisions including screwing a bone anchorage screw of each anchor assembly 7 into one or more adjacent pedicle portions of adjacent vertebrae in the spine, such that the anchor assemblies' receiver 5 mating parts align in a parallel plane within the subcutaneous fat layer of the back; 2) loading the support element 1 on top of the anchor assemblies including engaging the mated parts of the receiver 5 and the anchor assembly 7, either by sliding, snapping or otherwise positioning the support element 1 into the desired position; 3) accessing and locking the anchor assembly 7 in the support element 1 using the locking means feature of the anchor assembly 7 via the support element 1 apertures 6; and 4) optionally locking the bone anchorage screw feature of the anchor assembly 7 using the locking means feature for the bone anchorage screw via the support element 1 apertures 6.

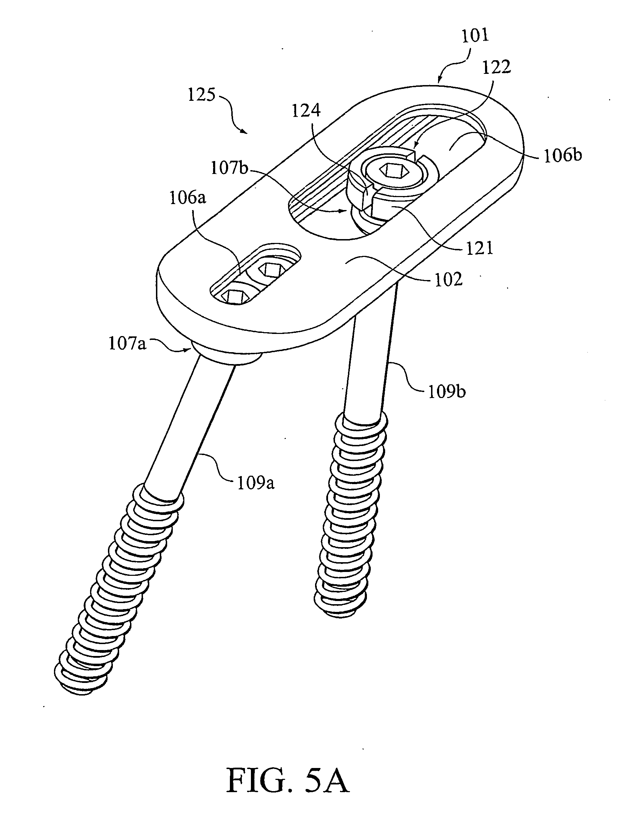

As shown in FIGS. 5A-E, FIG. 6 and FIG. 7, a support 101 is provided having a shape. The support 101 preferably is elongate and of a sufficient length to span a number of vertebrae in the spine. The support 101 shape can include board, elongated cross-section, rod, oval, square, and I-beam, as the needs of the patient's anatomy and/or mechanical requirements dictate. The length of the support 101 is minimally substantially the same length as required to span two or more vertebrae. In one implementation, the support 101 is substantially a length as required to span three vertebrae. In another implementation, the length of the support 101 is substantially between 25 to 140 millimeters. The support 101 can be made of materials that are durable and that can be implanted in a body, including titanium, stainless steel, carbon fiber, etc. In one implementation, the support 101 is made of titanium. In another implementation the support 101 is made of a biocompatible material, a reabsorbable material or a combination of any of the foregoing materials and/or alloys thereof. In use, the support 101 and attending anchor assemblies 107 (described in detail below) can be used for temporary or permanent implantation, with the degree of permanence determined post facto.

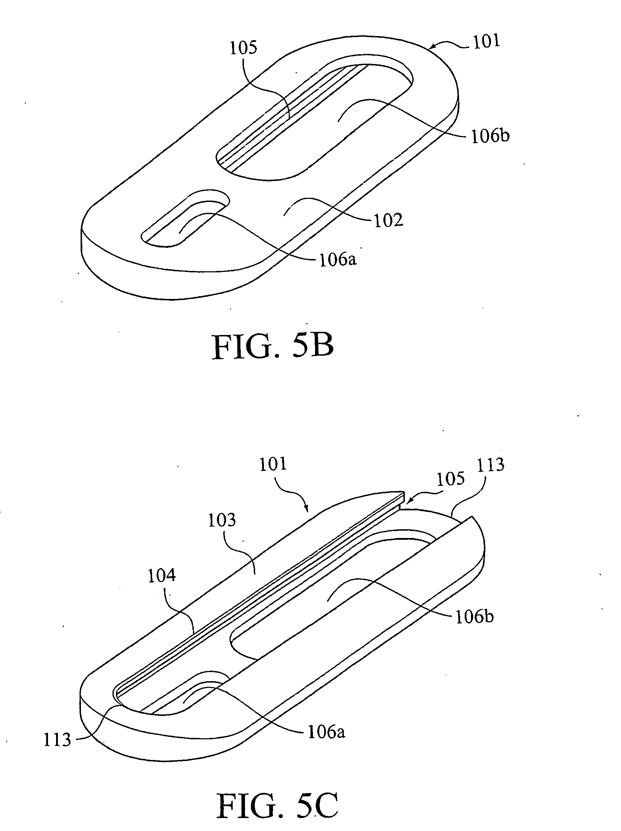

As shown in FIGS. 5B and 5C, the support 101 includes a top portion 102 and a bottom portion 103 having a bottom surface 104 and a receiver 105. Additionally, as shown in FIGS. 5A-D, FIG. 6, and FIG. 7, the support 101 can include a number of centrally aligned apertures 106. The apertures 106 may also be offset or staggered. The apertures 106 can have any of a number of shapes not limited to openings having a round, oval, scalloped or elongate shape. Any number of apertures 106 can be employed, including one or more. In use (i.e., assembled with a plurality of anchor assemblies 107) the support 101 top portion 102 can have a smooth surface with no protruding features (e.g., screw heads or the like).

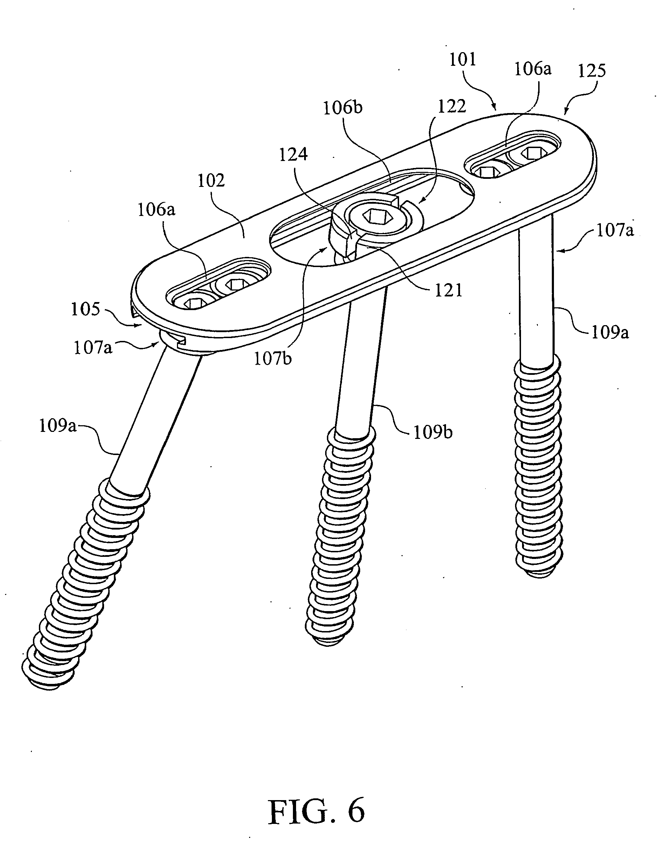

In one implementation, two apertures 106 per support 101 are included (see FIGS. 5A-D). In another implementation, three apertures 106 per support 101 are included (see FIG. 6). Any type of spacing of apertures 106 can be used, including even or staggered. In one implementation, the apertures 106 are spaced evenly and separated by a distance substantially equivalent to the spacing of the vertebrae of a spine. Alternatively, the apertures 106 can be spaced closer together than the spacing of vertebrae of a spine. The spacing of the apertures may also be determined by the type of bone to be treated, for example long bones such as the femur. In one implementation, the aperture(s) 6 are sized for and limited to a size sufficient for accessing the features of the anchor assembly 107 (described in detail below) with a tool or object through the top portion 102 of the support element 1 (e.g. to engage the locking means of the anchor assembly 107 using, for example, a hex-headed screw driver). In this way, as shown in FIG. 5A and FIG. 6, when the support 101 and the anchor assembly 107 are assembled, the base head 108 of the base 109 (both described in detail below), though accessible through the support 101, does not pass through the support 101.

In another implementation, the aperture(s) 106a are sized such that when the support 101 and the anchor assembly 107a are assembled, the base head 108 portion of the base 109 can engage the support 101 (e.g., from beneath), but the base 109 does not pass through the support 101.

In another implementation, the aperture(s) 106b are sized such that the anchor assembly 107b can pass completely through the support 101 top portion 102 or bottom portion 103, such that the anchor assembly 107b can be lockably engaged within the support 101 as desired.

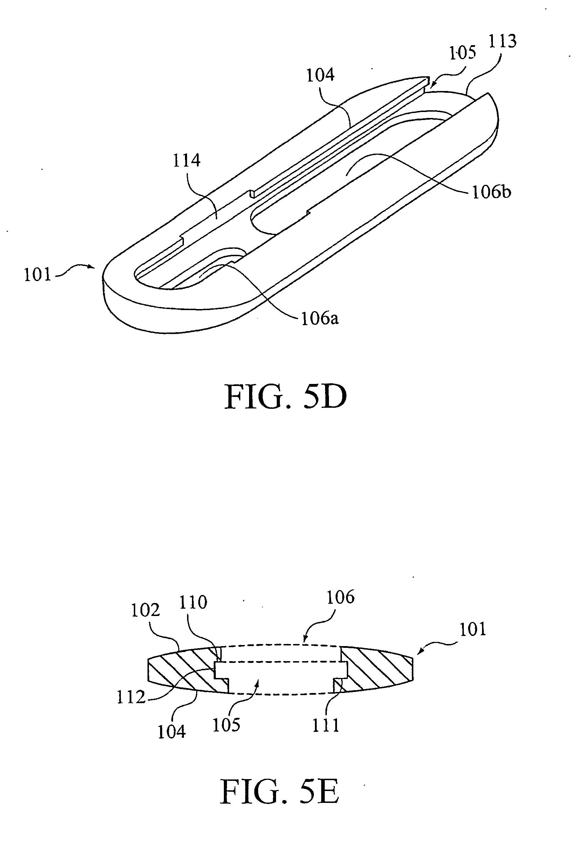

As shown in FIGS. 5B-E, the support 101 includes a receiver 105 that can be integrally disposed within the bottom surface 104 of the support 101. Alternatively, the receiver 105 can be coupled to the bottom surface 104 of the support 101 (for example, a track-type receiver 105 can be attached by rivets, screws or other attachment means, to the bottom surface 104 of the support 101). The receiver 105 can be configured as a slot, groove, track, dovetail, one-way snap-in design, or the like. In one implementation, the receiver 105 is configured in a twist-in configuration, wherein the anchor assembly 107 has two dimensions. The first dimension allows the anchor assembly 107 to pass into the receiver 105 (not shown). As the base head 108 is rotated 90 degrees with respect to the receiver 105 and, upon completing the 90-degree rotation, the second dimension maintains the base 109 in the receiver 105. In another implementation, as shown in FIGS. 5B-C and 5E, the receiver 105 is a T-slot configuration. As shown in FIG 5E, in cross-section, the receiver 105 includes a planar upper face 110, a planar lower face 111 and a planar medial face 112. Alternatively, where the configuration of the receiver 105 is a dovetail, the receiver 105 includes a planar upper face 110 and an angled face (not shown). In yet another alternative, where the receiver 105 is of a curved or rounded shape, the receiver 105 includes a curved or rounded receiver 105 face (not shown). The receiver 105 can span all or part of the length of the bottom surface 104 of the support 101. As shown in FIGS. 5C and 5D, each end 113 of the receiver 105 can be open or closed. In one implementation, both ends 113 of the receiver 105 are open (not shown). In another implementation, one end 113 of the receiver 105 is open and the opposite end 113 is closed (see FIGS. 5C and 5D). In another implementation, both ends 113 of the receiver 105 are closed (not shown).

As shown in FIG. 5D, the receiver 105 can include anchor assembly access ports 114 that provide openings in the pathway of the receiver 105, whereby the anchor assemblies 107 can be interconnected to the support 101 without having to traverse the length of the receiver 105 when accommodating multiple (or even a single) anchor assemblies 107. The anchor assembly access ports 114 can be of a sufficient size to accept the anchor assembly 107 and can be spaced evenly, or staggered as desired. The anchor assembly access ports 114 also allow assembly when the ends 113 of the receiver 105 are closed.

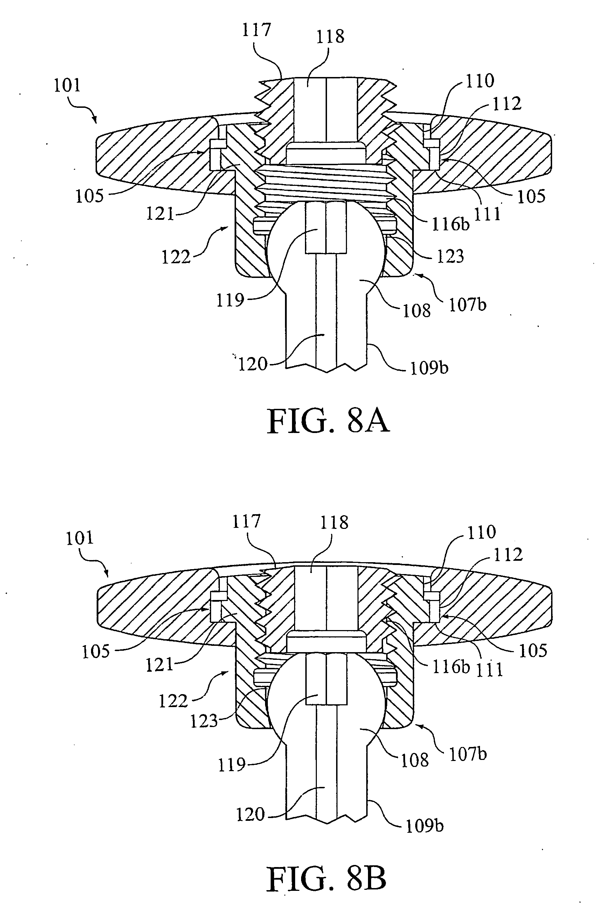

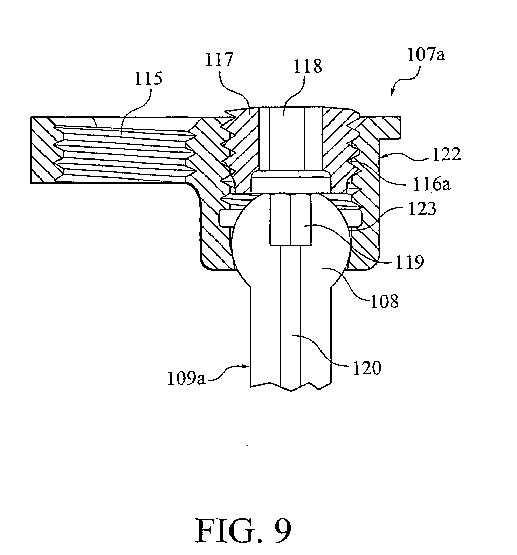

As shown in FIGS. 8A and 8B, the anchor assembly 107b is configured to interconnect with the shape of the receiver 105. As described above for the receiver 105, the anchor assembly 107 forms a complimentary shape, which can be any of a number of shapes. In some implementations, the mating between the support 101 and the anchor assembly 107 occurs only in two dimensions (e.g. where a 90 degree twisting receiver 105 is employed). As shown in FIGS. 8A-B and FIG 9, the anchor assembly 107b can be comprised of a head 122, a base 109b and a base head 108. The anchor assembly 107b can be comprised of numerous materials that are durable and that can be implanted in a body, including titanium, stainless steel, carbon fiber, etc. Additionally, the anchor assembly 107b can be comprised of a reabsorbable material or a biocompatible material, or a combination of any of the foregoing materials.

As shown in FIGS. 8A - and 8B, the anchor assembly 107b includes a means for locking the anchor assembly 107b to the support 101. In one implementation, as shown in FIG. 8A, where the receiver 105 and complimentary head 122 of the anchor assembly 107b are T-slot shaped, the means for locking the anchor assembly 107b can be a setscrew 117 threaded into the head 122 of the anchor assembly 107b, wherein the head 122 includes a threaded base aperture 116b and a deformable geometry. As shown in FIG. 8B, when the setscrew 117 is turned into the threaded base aperture 116b, the deformable head 121 is caused to splay outward such that the T-slot shape of the head 122 engages and locks against the receiver 105 planar medial face 112. Alternatively, the head 122 may additionally engage the receiver 105 planar upper face 110 or planer lower face 111 or both to effect locking. In one implementation, the means for locking the anchor assembly 107b is constructed and arranged such that a first step of turning the setscrew 117 into the threaded base aperture 116b results in engagement and tightening of the base head 108 within the anchor assembly receptacle 123 in a desired position. In this implementation, the means for locking is further constructed and arranged such that a second step of further turning the setscrew 117 results in an outward splaying of the deformable head 121, resulting in locking the anchor assembly 107b within the receiver 105 as discussed above. In another implementation, a cam can be substituted for the setscrew 117 to effect the locking of the anchor assembly 107b within the support 101 (not shown).

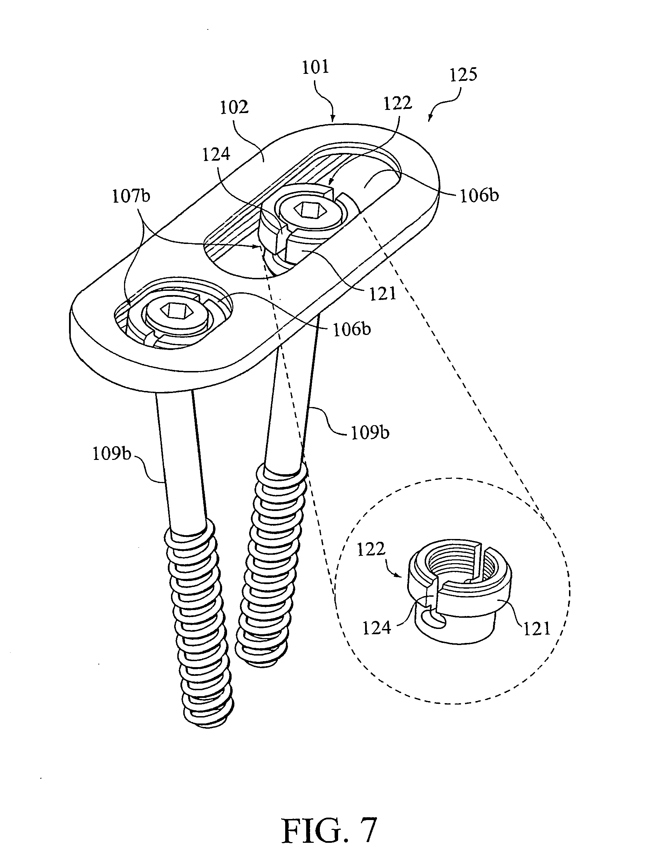

The deformable geometry of the deformable threaded base aperture 116b can be comprised of a void within the anchor assembly 107b head 122 wherein the void is selected from the list consisting of a cavity, slot notch, groove, cut out, gap and a recess. In one implementation, the void is tapered. In another implementation as shown in FIG. 5, FIG. 6, and FIG. 7, the void within the anchor assembly 107b head 122, can be a slot 124 cut into the head 122 (see detail in FIG. 7).

As shown in FIG. 9, the anchor assembly 107a includes an alternative means for locking the anchor assembly 107a to the support 101. In one implementation, where the receiver 105 and complimentary anchor assembly 107a are T-slot shaped, the means for locking the anchor assembly 107a can be a setscrew 117 disposed within a threaded anchor assembly 107a locker aperture 115 (see FIG. 9); wherein turning the setscrew 117 causes the setscrew 117 to move toward the top portion 102 of the support 101, where the setscrew 117 engages the receiver 105 planar upper face 110 (not shown). When the setscrew 117 engages the receiver 105, the anchor assembly 107a presses against the receiver 105 planer lower face 111 to effect locking (see FIG. 5). Alternatively, a cam can be substituted for the setscrew 117 to effect the locking of the anchor assembly 107a within the support 101.

In other implementations where the receiver 105 and complimentary anchor assembly 107 are of alternative shapes or configurations (e.g. dove tail or rounded), means analogous to those discussed above for locking an anchor assembly to the support are provided.

Other means for locking an anchor assembly 107 to the support 101 are envisioned, including means as disclosed in published U.S. Application Serial No. 2005 234,453 filed April 16, 2004, entitled "Subcutaneous Support".

As shown in FIGS. 8A-B and FIG. 9, the anchor assembly 107 includes a base 109 moveably disposed within the threaded base aperture 116a. The base 109 can be a screw, staple, hook or nail and of a type typically used for anchoring to a structure (e.g., to a bone). In one implementation, the base 109 is a screw of a type for insertion into the pedicle of a vertebra. In another implementation, the base can be attached to another bony structure.

Attachment of the base 109 to the anchor assembly 107 can be made in any of a number of ways. In one implementation, the attachment is through a hinge-type of connection between the base 109 and the anchor assembly 107 (not shown). In another implementation, as shown in FIGS. 8A-B and FIG. 9, the attachment is made between a polyaxial-type base head 108 on the base 109 and a complimentary receptacle 123 within the anchor assembly 107 head 122.

The anchor assembly 107 further includes a means for locking the base 109 within the anchor assembly 107 head 122. As shown in FIGS. 8A-B and FIG 9, for a polyaxial-type base head 108, the means for locking can include a setscrew 117 disposed within a threaded base aperture 116a. In this configuration, turning the setscrew 117 causes the setscrew 117 to press directly against the polyaxial base head 108 of the base 109, thereby forcing the polyaxial base head 108 against the receptacle 123 of the anchor assembly 107 to effect locking. Alternatively, where the base 109 is of the hinge-type, the means for locking could be comprised of a setscrew 117 disposed in a threaded base aperture 116a. In this configuration, turning the setscrew 117 causes the setscrew 117 to press directly against the base head 108 of the hinge-type base 109, thereby creating friction against the hinge's pin to effect locking (not shown). In another implementation, a cam can be substituted for the setscrew 117 to effect locking (not shown).

As shown in FIGS. 8A-B and FIG. 9, another implementation of the anchor assembly 107 includes a longitudinal aperture 120 through the base 109 and base head 108, a tool interface 119 and a setscrew aperture 118. The longitudinal aperture 120 and setscrew aperture 118 are configured such that an instrument, wire (e.g. a K-wire) or other guide can pass through the entire anchor assembly 107. The longitudinal aperture 120 and setscrew aperture 118 can also be configured for delivery of a bone substitute into the bone to be treated. The setscrew aperture 118 is further configured such that a tool or instrument can pass through the setscrew aperture 118 to engage the tool interface 119 of the base 109. Alternatively, the setscrew 117 can be a cam (not shown).

The setscrew aperture 118 can be any shape and can be sized to accommodate the through passage and use of objects and tools without affecting the positioning of the setscrew. The setscrew aperture 118 and longitudinal aperture 120 can also be sized and configured to allow passage of instruments (for example, expandable structures, bone cutting or scraping tools, tools for delivery of bone substitute materials) used for treating and fixing a fracture of the bone, such as in a kyphoplasty procedure.

The longitudinal aperture 120 can have any desired cross-sectional shape including but not limited to round, square, hexagonal, oval or any regular or irregular shape.

The tool interface 119 can be any shape suitable for receiving a tool for manipulating the base 109. For example, where the base 109 is a screw, the tool interface 119 can be a hex shape, or any other commonly used screw head tool interface shape.

Where an anchor assembly 107 has the above configuration, the setscrew 117 can be pre-positioned within the base aperture 116a without being tightened. The setscrew aperture 118 and longitudinal aperture 120 (passing through the base 109 and base head 108) enable access through a pre-assembled implementation of the anchor assembly 107. Additionally, wherein the anchor assembly 107 is pre-assembled, access is provided to the tool interface 119 of the base head 108 through the setscrew aperture 118.

A method of using an anchor assembly 107 having the above configuration to secure the anchor assembly 107 into a structure (e.g. into a bone, such as a vertebra) can include the steps of: 1) positioning a K-wire at the target site; 2) passing the anchor assembly 107 onto the K-wire and guiding the anchor assembly 107 down the K-wire length to the target site; 3) engaging the tool interface 119 of the base 109 by passing through the setscrew aperture 118 with a tool; 4) securing the base 109 into the structure at the target site using the tool (e.g. by screwing into vertebrae), 5) withdrawing the tool from the tool interface 119; and 6) securing the base 109 or support 101 to the anchor assembly 107 using the setscrew 117. Optionally, the method can include treating the structure to be secured.

The support 101 and one or more anchor assemblies 107, once assembled, can be used to support a bony structure. When mated, the support 101 and one or more anchor assemblies 107 form a support assembly 125 (see FIG 5A, FIG. 6 and FIG 7). The bony structure supported can include a femur or other bones of the leg (e.g. tibia and fibula), bones of the arm and wrist (e.g. humerus, radius and ulna), and other bones such as the calcaneus, pelvis, spine and the like. Support can be provided for a single bone (i.e. a long bone such as the femur, tibia, humerus) or for more than one bone (i.e. vertebrae).

In use, the support assembly 125 can support a bony structure wherein the support 101 is disposed within a body location including the subcutaneous fat layer of the back, muscle, cartilage, bone and the like. Alternatively, the support 101 is disposed adjacent to bone. In another implementation, the support 101 is disposed external to the body.

Additionally, the support assembly 125 includes a freedom of movement with regard to the base 109 within the anchor assembly 107 and the anchor assembly 107 within the support 101. That is, prior to locking respective base 109 and the anchor assembly 107 elements of the support assembly 125 (hereinafter referred to as an unlocked configuration), the elements of the support assembly 125 are movable and have one or more degrees of freedom so as to allow for movement of the underlying structure being supported. For example, in the unlocked configuration, the support assembly 125 is configurable so as to facilitate manipulation of vertebral spacing and/or curvature correction.

As shown in FIG 5A, FIG. 6 and FIG 7, several implementations of the support assembly 125 can be useful to effect an increase or decrease the vertebral disc space/height, or to increase or decrease the amount or lordotic/kyphotic curve of the spine, also called curvatures of the spine. Manipulation of vertebral disc spacing and spine curvature can be achieved with each of the implementations shown.

As shown in FIG. 5, one implementation of support assembly 125 includes a support 101 with a first and a second anchor assembly 107. In this implementation the first anchor assembly 107a includes independent base 109 -to-anchor assembly 107 and anchor assembly 107 -to-support 101 locking means, and is configured such that when assembled with the support 101, the base 109a does not pass through the aperture 106a. In contrast, the second anchor assembly 107b is configured for substantially concurrent base 109 and anchor assembly 107 locking and is configured to be passable through the support 101. As shown in FIGS. 5A-E, the sizing of the aperture 6, determines whether a first or second anchor assembly 107 variant (107a or 107b) can be accommodated.

As shown in FIG. 6, in another implementation, an additional first anchor assembly 107a, can be arranged such that the second anchor assembly 107b is flanked by two first anchor assemblies 107a. In use, this arrangement as shown in FIG. 2, can provide attachment to and manipulation of three consecutive vertebrae. For example, the flanking first anchor assemblies 107a could be attached to two individual vertebrae that flank a third vertebrae to which the second anchor assembly 107b could be attached.

As shown in FIG. 7, in another implementation, the support assembly 125 can include two anchor assemblies 107b configured for substantially concurrent base 109 and anchor assembly 107 locking and configured to be passable through the support 101.

A method of use of the device according to the invention for effecting a desired vertebral disc spacing, can include the steps of: 1) implanting the bases 109 of a plurality of anchor assemblies 107 into vertebrae, wherein the bases 109 of the anchor assemblies 107 are unlocked within the anchor assemblies 107 for free movement of the head 122; 2) interconnecting the anchor assemblies 107 with the receiver 105 of the support 101, wherein the anchor assemblies 107 are unlocked within the receiver 105; 3) locking the bases 109 within the anchor assemblies 107 (e.g. using a setscrew 117 or cam); 4) compressing or distracting the bases 109 in relation to each other (e.g. to achieve a parallel displacement of the instrumented vertebrae ("Instrumented" meaning where a physical connection exists between a structure (e.g. a vertebra) and a medical device or instrument)); and 5) locking the anchor assemblies 107 within the support 101 (e.g. using a set screw 17 or cam).

A method of use of the device according to the invention for effecting a desired curvature of the spine can include the steps of: 1) implanting the bases 109 of a plurality of anchor assemblies 107 into vertebrae, wherein the bases 109 of the anchor assemblies 107 are unlocked within the anchor assemblies 107 for free movement of the head 122; 2) interconnecting the anchor assemblies 107 with the receiver 105 of the support 101, wherein the anchor assemblies 107 are unlocked within the receiver 105; 3) compressing or distracting the anchor assemblies 107 in relation to each other (e.g. to affect the lordotic/kyphotic curvature of the spine); 4) locking the bases 109 within the anchor assemblies 107 and locking the anchor assemblies 107 within the support 101 (e.g. using a setscrew 117 or cam).

Another method of using the device according to the invention to support the spine can include the steps of: 1) setting a series of anchor assemblies 107 percutaneously in place along the spine through a series of small incisions including screwing a bone anchorage screw of each anchor assembly 107 into one or more adjacent pedicle portions of adjacent vertebrae in the spine, such that the anchor assemblies' receiver 105 mating parts align in a parallel plane within the subcutaneous fat layer of the back; 2) loading the support 101 on top of the anchor assemblies 107 including engaging the mated parts of the receiver 105 and the anchor assembly 107, either by sliding, snapping or otherwise positioning the support 101 into the desired position; 3) accessing and locking the anchor assembly 107 in the support 101 using the locking means of the anchor assembly 107 via the support 101 apertures 106; and 4) optionally locking the base 109 of the anchor assembly 107 using the locking means (e.g. setscrew 117 or a cam) for the bone anchorage screw via the support 101 apertures 106.

Other methods of using one aspect of the invention to support the spine can include a variety of combinations of the two types of anchor assembly (107a and 107b) described herein and shown in FIG 5A, FIG. 6 and FIG 7. A number of combinations of the two types of anchor assembly 107 including a support 101 having two or three anchor assemblies 107 attachable thereto can be used to support the spine. For example, the support assembly 125 could include two anchor assemblies 107a, or two anchor assemblies 107b (as shown in FIG. 7), or one anchor assembly 107a and one anchor assembly 107b (as shown in FIG 5A). Alternatively, the support assembly 125 could include three anchor assemblies 107 consisting of two anchor assemblies 107a and one anchor assembly 107b arranged in the support in any possible substantially linear order. An example of one such arrangement is shown in FIG. 6. Alternatively, the support assembly 125 could include three anchor assemblies 107 consisting of two anchor assemblies 107b and one anchor assembly 107a arranged in the support in any possible substantially linear order (not shown).

Additional permutations for using the device according to the invention include employing various alternatively ordered steps for locking anchor assemblies 107 within the support 101 and for locking the base 109 within the anchor assembly 107. For example, a given anchor assembly 107a can be locked in position within the support 101 in a first step, followed by locking of the base 109 within the anchor assembly 107a or vice versa.

The methods of supporting the spine can also be used in conjunction with a kyphoplasty procedure. Kyphoplasty is a percutaneous technique involving the use of an expandable structure, such as a balloon catheter, to create a cavity or void within the vertebral body, followed by filling the cavity with a bone substitute to form an "internal cast". The bone substitute could be any appropriate filling material used in orthopedic surgery, including but not limited to, allograft or autograft tissue, hydroxyapatite, epoxy, PMMA bone cement or synthetic bone substitutes, medical grade plaster of Paris or calcium phosphate or calcium Sulfate cements. Methods and instruments suitable for such treatment are more fully described in U.S. Pat. Nos. 4,969,888 and 5,108,404. Kyphoplasty can be used to reduce vertebral compression fractures and to move bone with precision, thus restoring as close to normal the pre-fracture anatomy of the vertebral body. Vertebral compression fractures caused by trauma (for example, due to automobile accidents or falls), have traditionally been treated with open reduction, internal fixation stabilization hardware and fusion techniques using a posterior approach. The stabilization hardware is used to offload the fractured vertebral body and to stop motion across the disc so that bone graft can fuse one vertebra to the next and the stabilization hardware usually becomes a permanent implant, In trauma, the stabilization hardware may be designed to facilitate easy removal after fusion has occurred. Stabilization hardware can take many forms, including those described herein.

The combination of kyphoplasty and insertion of stabilization hardware utilizing the naturally occurring interior muscle plane as described in Wiltse and Spender, Spine (1988) 13(6):696-706, satisfies the goals of improving the quality of patient care through minimally invasive surgical therapy.

## Claims

1. A bone support apparatus comprising
a support element (1), which is configured for implantable support of bone, has a top portion (2), a bottom portion (3) and one or more apertures (6) passing therethrough, the bottom portion (3) having a bottom surface (4) comprising a receiver (5) configured to receive a plurality of anchor assemblies (7);
more than one anchor assemblies (t); and one base (9) for each anchor assembly (7), each; **characterized in that** base (9) being configured to be implanted into bone, and having a head (8) each of the anchor assemblies (7) comprises:
a means for locking the anchor assembly (7) to the bottom: portion (3) of the support element (1), the means for locking comprising a locker aperture (15); and
means for locking each base (9) to the corresponding anchor assembly (7) such that, when assembled, the base (9) does not pass: through the support element (1).

2. The apparatus of claim 1, wherein the bone supported is selected from the group consisting of a spine, femur, tibia, fibula, humerus, radius, ulna, calcaneous, and a pelvis.

3. The apparatus of claim 1, wherein the base head (8) is movably disposed within the anchor assembly (7).

4. The apparatus of claim 1, wherein the one or more apertures (6) have a dimensional configuration providing access to the base (9) and the means for locking the base to the anchor assembly (7) through the top portion (2) of the support element (1).

5. The apparatus of claim 1, wherein the support element (1) has a shape selected from the group consisting of a board, plate, elongated cross-section, oval, square, I-beam and a rod.

6. The apparatus of claim 1, wherein the support element (1) is sized to substantially span two or more vertebrae.

7. The apparatus of claim 1, wherein the receiver (5) is integrally disposed within the bottom surface (4) of the bottom portion (3) of the support element (1).

8. The apparatus of claim 1, wherein the receiver (5) is attached to the bottom surface (4) of the bottom portion (3) of the support element (1).

9. The apparatus of claim 1, wherein the receiver (5) is comprised of a plurality of access ports sized for coupling the anchor assembly (7) to the receiver (5) distally from the receiver ends (13).

10. The apparatus of claim 1, wherein the receiver (5) and the anchor assemblies (7) are configured in an interconnecting geometry comprising a T-slot, wherein the T-slot of the receiver (5) comprises a planar upper face (10), and a planar lower face (11), and
wherein the means for locking the anchor assemblies (7) to the support element (1) includes
a setscrew (17) disposed within the locker aperture (15);
wherein the setscrew (17) and locker aperture (15) are threaded so as to lockably engage a receiver planar upper face (10) upon turning; and
wherein upon so engaging a receiver planar upper face (10), the setscrew (17) causes the anchor assembly (7) to press against the receiver lower planar face (11) to effect locking.

11. The apparatus of claim 1, wherein the means for locking the anchor assembly (7) to the support element (1) is comprised of a threaded blind aperture (18) having a slot (19) substantially aligned longitudinally with the receiver (5) thereby providing expandable walls (22), a floor (20) having a cut channel (21) therethrough and a setscrew (17); and
wherein turning the setscrew (17) into the blind aperture (18) causes the expandable walls (22) to expand outwardly;
wherein the walls (22) engage a receiver planar medial surface to effect locking.

12. The apparatus of claim 1, wherein the base (9) is selected from the group consisting of a screw, staple, nail, hook and a pin.

13. The apparatus of claim 1, wherein a second plurality of anchor assemblies are passable through one of the apertures (6) and lockably engagable with the support element (1).

14. The apparatus of claim 1, wherein the one or more apertures (6) have a dimensional configuration providing access to a base (9) and the means for locking an anchor assembly (7) when assembled with the support element (1).

15. The apparatus of claim 1, wherein the means for locking the anchor assemblies (7) to the support element (1) includes a deformable geometry of the head portion (121) of the one or more anchor assemblies (107b).

16. The apparatus of claim 15, wherein the deformable geometry of the head (121) is comprised of a tapered void within the head, and a setscrew (117);
wherein turning the setscrew (117) into the threaded base aperture (116b) results in deformation of the head (121) outwardly; and
wherein the head (121) engages the receiver (105) to effect locking.

## Patentansprüche

1. Knochenstützvorrichtung, aufweisend
ein zur implantierbaren Stütze von Knochen ausgestaltetes Stützelement (1) mit einem oberen Abschnitt (2), einem unteren Abschnitt (3) und einer oder mehreren dort hindurchführenden Öffnungen (6), wobei der untere Abschnitt (3) eine untere Fläche (4) aufweist, die eine zur Aufnahme einer Vielzahl von Verankerungselementen (7) ausgestaltete Aufnahme (5) umfasst;
mehr als ein Verankerungselement (7); und
eine Basis (9) für jedes Verankerungselement (7), wobei jede Basis (9) zur Implantation in einen Knochen ausgestaltet ist und einen Kopf (8) aufweist;
**dadurch gekennzeichnet, dass**
jedes der Verankerungselemente (7) aufweist:
ein Mittel zur Arretierung des Verankerungselements (7) an dem unteren Abschnitt (3) des Stützelements (1), wobei das Mittel zur Arretierung eine Arretieröffnung (15) umfasst; und
Mittel zur Arretierung jeder Basis (9) an dem entsprechenden Verankerungselement (7), so dass im montierten Zustand die Basis (9) nicht durch das Stützelement (1) hindurch tritt.

2. Vorrichtung nach Anspruch 1, wobei der gestützte Knochen aus der aus Wirbelsäule, Oberschenkel, Schienbein, Wadenbein, Oberarm, Speiche, Elle, Fersenbein und Becken bestehenden Gruppe ausgewählt ist.

3. Vorrichtung nach Anspruch 1, wobei der Basiskopf (8) innerhalb des Verankerungselements (7) bewegbar angeordnet ist.

4. Vorrichtung nach Anspruch 1, wobei die eine oder die mehreren Öffnungen (6) eine räumliche Anordnung aufweisen, die einen Zugang zu der Basis (9) und zu dem Mittel zur Arretierung der Basis an dem Verankerungselement (7) durch den oberen Abschnitt (2) des Stützelements (1) hindurch bereitstellen.

5. Vorrichtung nach Anspruch 1, wobei das Stützelement (1) eine Form aufweist, die aus der aus einer Platine, einer Platte, einem länglichen Profil, einem Oval, einem Viereck, einem I-Profil und einer Stange bestehenden Gruppe ausgewählt ist.

6. Vorrichtung nach Anspruch 1, wobei das Stützelement (1) dimensioniert ist, um im Wesentlichen zwei oder mehrere Wirbel zu überspannen.

7. Vorrichtung nach Anspruch 1, wobei der Aufnehmer (5) innerhalb der unteren Fläche (4) des unteren Abschnitts (3) des Stützelements (1) integral angeordnet ist.

8. Vorrichtung nach Anspruch 1, wobei der Aufnehmer (5) an der unteren Fläche (4) des unteren Abschnitts (3) des Stützelements (1) angebracht ist.

9. Vorrichtung nach Anspruch 1, wobei der Aufnehmer (5) eine Vielzahl von Zugangsöffnungen aufweist, die zur Verbindung des Verankerungselements (7) an dem Aufnehmer (5) distal von den Aufnehmerenden (13) dimensioniert sind.

10. Vorrichtung nach Anspruch 1, wobei der Aufnehmer (5) und die Verankerungselemente (7) in einer miteinander verbundenen geometrischen, einen T-Schlitz aufweisenden Anordnung konfiguriert sind, wobei der T-Schlitz des Aufnehmers (5) eine planare obere Fläche (10) und eine planare untere Fläche (11) aufweist, und
wobei das Mittel zur Arretierung der Verankerungselemente (7) an dem Stützelement (1) aufweist:
eine innerhalb der Arretierungsöffnung (15) angeordnete Stellschraube (17);
wobei die Stellschraube (17) und die Arretierungsöffnung (15) ein Gewinde aufweisen, um die planare obere Fläche (10) des Aufnehmers beim Drehen arretierbar in Eingriff zu bringen; und
wobei bei einem solchen in Eingriffbringen einer planaren oberen Fläche (10) des Aufnehmers die Stellschraube (17) bewirkt, dass das Verankerungselement (7) gegen die untere planare Fläche (11) des Aufnehmers drückt, um eine Arretierung herbeizuführen.

11. Vorrichtung nach Anspruch 1, wobei das Mittel zur Arretierung des Verankerungselements (7) an dem Stützelement (1) ein Gewindeblindloch (18) mit einem Schlitz (19) aufweist, der im wesentlichen in Längsrichtung zu dem Aufnehmer (5) ausgerichtet ist, wodurch erweiterbare Wände (22) bereitgestellt werden, wobei ein Boden (20) eine Schnittführung (21) dort hindurch und eine Stellschraube (17) aufweist; und
wobei ein Drehen der Stellschraube (17) in das Blindloch (18) hinein bewirkt, dass sich die erweiterbaren Wände (22) nach außen hin erweitern;
wobei die Wände (22) in eine planare Mittelfläche des Aufnehmers eingreifen, um eine Arretierung herbeizuführen.

12. Vorrichtung nach Anspruch 1, wobei die Basis (9) aus der aus einer Schraube, einer Öse, einem Nagel, einem Haken und einem Stift bestehenden Gruppe ausgewählt ist.

13. Vorrichtung nach Anspruch 1, wobei eine zweite Vielzahl von Verankerungselementen durch eine der Öffnungen (6) hindurchführbar ist und mit dem Stützelement (1) arretierbar in Eingriff bringbar ist.

14. Vorrichtung nach Anspruch 1, wobei die eine oder die mehreren Öffnungen (6) eine räumliche Anordnung aufweisen, die einen Zugang zu der Basis (9) und zu dem Mittel zur Arretierung eines Verankerungselementes (7) beim Zusammenbau mit dem Stützelement (1) bereitstellen.

15. Vorrichtung nach Anspruch 1, wobei das Mittel zur Arretierung der Verankerungselemente (7) an dem Stützelement (1) eine verformbare Geometrie des Kopfabschnitts (121) des einen oder der mehreren Verankerungselemente (107b) aufweist.

16. Vorrichtung nach Anspruch 15, wobei die verformbare Geometrie des Kopfes (121) einen sich verjüngenden Hohlraum innerhalb des Kopfes und eine Stellschraube (117) aufweist;
wobei ein Drehen der Stellschraube (117) in eine mit einem Gewinde versehene Basisöffnung (116b) zu einer Verformung des Kopfes (121) nach außen hin führt; und
wobei der Kopf (121) in den Aufnehmer (105) eingreift, um eine Arretierung zu herbeizuführen.

## Revendications

1. Un appareil pour support d'os comprenant,
- un élément de support (1), lequel est configuré pour le support implantable d'os, une partie supérieure (2), une partie inférieure (3) et une ou plusieurs ouvertures (6) passant au travers des parties, la partie inférieure (3) ayant une surface inférieure (4) comprenant un logement (5) configuré pour recevoir une pluralité d'ensembles d'ancrages (7); plus que un ensemble d'ancrage (7); et une base (9) pour chaque ensemble d'ancrage (7), chaque base (9) étant configurée pour être implantée dans l'os et ayant une tête (8);
**caractérisé en ce que**
chacun des ensembles d'ancrages (7) comprend :
- un moyen pour bloquer l'ensemble d'ancrage (7) dans la partie inférieure (3) de l'élément de support (1), le moyen de blocage comprenant une ouverture de blocage (15) ; et
- des moyens pour bloquer chaque base (9) à l'ensemble d'ancrage correspondant (7) de sorte que, lors de l'assemblage, la base (9) ne passe pas au travers de l'élément de support (1).

2. Un appareil selon la revendication 1, dans lequel l'os soutenu est choisi parmi le groupe constitué par la colonne vertébrale, le fémur, le tibia, le péroné, l'humérus, le radius, le cubitus, le calcanéus, et le pelvis.

3. Un appareil selon la revendication 1, dans lequel la tête (8) de la base est disposée de façon mobile dans l'ensemble d'ancrage (7).

4. Un appareil selon la revendication 1, dans lequel une ou plusieurs ouvertures (6) ont une configuration de dimension fournissant l'accès à la base (9) et aux moyens pour bloquer la base à l'ensemble d'ancrage (7) via la partie supérieure (2) de l'élément de support (1).

5. Un appareil selon la revendication 1, dans lequel l'élément de support (1) a une forme sélectionnée à partir du groupe constitué par une planche, une plaque, une coupe transversale de forme allongée, un ovale, un carré, un barreau en forme de I et une tige.

6. Un appareil selon la revendication 1, dans lequel l'élément de support (1) est dimensionné pour porter substantiellement deux ou plusieurs vertèbres.

7. Un appareil selon la revendication 1, dans lequel le logement (5) est intégralement disposé à l'intérieur de la surface inférieure (4) de la partie inférieure (3) de l'élément de support(1).

8. Un appareil selon la revendication 1, dans lequel le logement (5) est joint à la surface inférieure (4) de la partie inférieure (3) de l'élément de support (1).

9. Un appareil selon la revendication 1, dans lequel le logement (5) est composé d'une pluralité d'ouvertures d'accès dimensionnées pour coupler l'ensemble d'ancrage (7) au logement (5) de manière distal par rapport à l'extrémité (13) du logement.

10. Un appareil selon la revendication 1, dans lequel le logement (5) et les ensembles d'ancrages (7) sont configurés dans une géométrie d'interconnexion comprenant une rainure en forme de T, dans lequel la rainure en forme de T du logement (5) comprend une face plane supérieure (10), et une face plane inférieure (11) et
dans lequel le moyen pour bloquer les ensembles d'ancrages (7) à l'élément de support (1) comprend
une vis de pression (17) disposée dans l'ouverture de blocage (15) ;
dans lequel la vis de pression (17) et l'ouverture de blocage (15) sont filetées de manière à se mettre en prise de façon bloquante avec la face plane supérieure (10) du logement par rotation ; et
dans lequel une fois la face plane supérieure (10) du logement mise en prise, la vis de pression (17) provoque que l'ensemble d'ancrage (7) appuie sur la face plane inférieure du logement (11) pour effectuer le blocage.

11. Un appareil selon la revendication 1, dans lequel le moyen pour bloquer l'ensemble d'ancrage (7) à l'élément de support (1) est composé d'une ouverture borgne filetée (18), ayant une fente (19) sensiblement alignée longitudinalement avec le logement (5) fournissant ainsi des parois extensibles (22), un planché (20) ayant un canal découpé (21) à travers celui-ci et une vis de pression (17) ; et
dans lequel la rotation de la vis de pression (17) dans l'ouverture borgne (18) provoque l'extension des parois extensibles (22) vers l'extérieur;
dans lequel les parois (22) se mettent en prise avec une surface plane médiane du logement pour effectuer le blocage.

12. Un appareil selon la revendication 1, dans lequel la base (9) est choisie parmi le groupe constitué par une vis, une agrafe, un clou, un crochet et une broche.

13. Un appareil selon la revendication 1, dans lequel une seconde pluralité d'ensembles d'ancrages peuvent passer au travers d'une des ouvertures (6) et peuvent être bloquées en étant en prise avec l'élément de support (1).

14. Un appareil selon la revendication 1, dans lequel une ou plusieurs ouvertures (6) ont une configuration de dimension fournissant un accès à la base (9) et au moyen pour bloquer un ensemble d'ancrage (7) lorsqu'il est assemblé avec l'élément de support (1).

15. Un appareil selon la revendication 1, dans lequel le moyen de blocage des ensembles d'ancrages (7) à l'élément de support (1) comprend une géométrie déformable de la partie de tête (121) d'un ou plusieurs ensembles d'ancrages (107 b).

16. Un appareil selon la revendication 15, dans lequel la géométrie déformable de la tête (121) est composée d'un vide conique dans la tête et d'une vis de pression (117) ;
dans lequel la rotation de la vis de pression dans l'ouverture de base filetée (116b) provoque une déformation de la tête (121) extérieurement ; et
dans lequel la tête (121) s'engage dans le logement (105) pour effectuer le blocage.
